# EUROPEAN PATENT APPLICATION

(11) **EP 3 692 994 A1**
(43) Date of publication of application: **12.08.2020**
(21) Application number: 18866348.8
(22) Date of filing: 14.08.2018
(51) Int. Cl.: A61K 31/569, A61P 7/00, A61P 35/00

(54) **FORMULATION CONTAINING A-DECARBONIZED-5 ANDROSTANE COMPOUND FOR INCREASING WHITE BLOOD CELL AND USE THEREOF**

(30) Priority: 13.10.2017 CN 201710953300
(71) Applicant: Shanghai Ao Qi Medical Technology Co., Ltd., Shanghai 200002 (CN)
(72) Inventor: CHEN, Yajun, Shanghai 200002 (CN); CHEN, Zhihua, Shanghai 200002 (CN); WANG, Wenya, Shanghai 200002 (CN)
(74) Representative: Di Giovine, Paolo
(86) International application number: PCT/CN2018/100430
(87) International publication number: WO 2019/072014

(57) **Abstract**

A formulation containing an A-decarbonized-5a-androstane compound for increasing white blood cells and use thereof. The A-decarbonized-5α-androstane compound can prevent, alleviate and improve the reduction in the number of white blood cells caused by chemotherapy or radiation therapy, and can be used for preparing a preparation or a composition, the preparation or the composition being used for (a) increasing the number of white blood cells or (b) preventing or treating leucopenia.

## Description

### FIELD OF THE INVENTION

The present invention relates to the field of medicine and specifically, relates to a formulation containing A-decarbonized-5α-androstane compound for increasing white blood cell and use thereof

### BACKGROUND OF THE INVENTION

Leukopenia is a disease that the number of leukocytes in human peripheral blood is continuously below 4.0x10⁹/L. Leukopenia is usually caused by drugs, radiation or other chemical poisons. Leukopenia can cause symptoms such as low immunity, loss of appetite, and limb weakness, etc.

At present, chemotherapy and radiotherapy are commonly used in the treatment of tumors. However, chemotherapy and radiotherapy often lead to leukopenia. While killing cancer cells, they also damage normal tissues, and in particular, most significantly inhibit bone marrow hematopoietic function, thereby resulting in continuous reduction of peripheral leukocytes. This condition not only hinders the continuous progress of chemotherapy and radiotherapy, but also causes serious consequences.

Currently, some medicines for increasing white blood cell are often used in clinic to increase the number of white blood cells in patients' blood, so that radiotherapy and chemotherapy can be conducted smoothly as scheduled and the periodic treatment of radiotherapy and chemotherapy can be ensured. The medicines for increasing white blood cell mainly comprise (1) Chinese herbal medicines, such as *Panax ginseng, Astragalus mongholicus, Codonopsis pilosula,* fruit of *Ligustrum lucidum,* stem of *Spatholobus suberectus Dunn,* fruit of *Lycium chinensis, Rehmannia glutinosa (Gaetn.) Libosch,* etc; (2). Chinese medicine compound preparation, such as YuluTang, BaoyuanTang, LiuweiDihuang oral liquid, Shengbai Pill, WujiBaifeng Pill, JianpiYishen Granules, Chang'anShengbai Granules, Shengbai Tablets, Shenqi Tablets, YangxueShengbai Capsules, etc; (3). Chemical medicines, such as leucogen, batiloli, vitamin B, berbmine hydrochloride, inosine, DNA, etc; and (4). Biological preparation, such as colony stimulating factor (CSF), granulocyte colony stimulating factor (G-CSF), granulocyte-macrophage colony stimulating factor (GM-CSF), etc. Among them, the effects of the first three types of Chinese and Chemical medicines are relatively limited. Although the hematopoietic stimulating factors have significant effects, they have short active time and a relatively fluctuating effect, cause obvious side-effects, are expensive, and have a large financial burden on patients.

Therefore, there is an urgent need in the art to develop a medicine for increasing white blood cells in clinic with significant efficacy, small side effects and convenient use.

### SUMMARY OF THE INVENTION

The purpose of the present invention is to provide a medicine for increasing white blood cells with significant effect, small side effects and convenient use.

In the first aspect of the present invention, it provides a use of an A-decarbonized-5α-androstane compound for manufacturing a preparation or a composition, wherein the preparation or the composition is used for (a) increasing the number of white blood cells; or (b) preventing or treating leucopenia.

In another preferred embodiment, the A-decarbonized-5α-androstane compound has a structure of formula I: 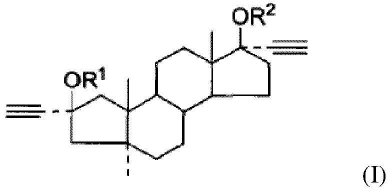 wherein,
R¹ and R² are independently selected from the group consisting of H, substituted or unsubstituted C₁₋₁₀ alkyl, substituted or unsubstituted C₃₋₈ cycloalkyl, substituted or unsubstituted benzene ring (or phenyl), substituted or unsubstituted benzoyl, substituted or unsubstituted COCₙH₂ₙ₊₁, substituted or unsubstituted COCᵣH₂ᵣCOOCₘH₂ₘ₊₁, or -COCₚH₂ₚCOO-W; wherein each of n, p, r, and m is independently an integer from 0 to 18, W is H, Na⁺, K⁺, NH₄⁺, 1/2Ca²⁺, 1/2Mg²⁺, 1/2(AlOH)²⁺ or 1/2Zn²⁺,
the term "substituted" means a substitution with one or more (such as 1-3) substituents selected from the group consisting of hydroxyl, halogen, nitro, amino, amine and carboxyl.

In another preferred embodiment, the A-decarbonized-5α-androstane compound is selected from the group consisting of:
2α,17α-diethynyl-A-decarbonized-5α-androstane-2β,17β-dihydroxyl compound;
2α,17α-diethynyl-A-decarbonized-5α-androstane-2β, 17β-dihydroxyl diacetate compound;
2α,17α-diethynyl-A-decarbonized-5α-androstane-2β, 17β-dihydroxyl dipropionate compound;
2α,17α-diacetyl-A-decarbonized-5α-androstane-2β, 17β-dihydroxyl-2β-monosuccinate compound;
2α,17α-diethynyl-A-decarbonized-5α-androstane-2β,17β-bissuccinate compound;
2α,17α-diethynyl-A-decarbonized-5α-androstane-2β,17β-dibutyrate compound;
2α,17α-dihydroxylpropynyl-A-decarbonized-5α-androstane-2β,17β-dihydroxyl compound;
2α,17α-dicyano-A-decarbonized-5α-androstane-2β,17β-dihydroxyl compound;
2α,17α-diethynyl-A-decarbonized-5α-androstane-2β, 17β-dihydroxyl ditrichloroacetate compound;
2α,17α-diethynyl-A-decarbonized-5α-androstane-2β,17β-dihydroxyl-2β-propionate-17β-su ccinate compound;
2α,17α-dipropynyl-A-decarbonized-5α-androstane-2β, 17β-dihydroxyl compound; and/or
2α,17α-dipropynyl-A-decarbonized-5α-androstane-2β, 17β-dihydroxyl dipropionate compound.

In another preferred embodiment, the composition is a pharmaceutical composition, a dietary supplement composition, or a healthcare composition.

In another preferred embodiment, the composition comprises (a) an A-decarbonized-5α-androstane compound; and (b) a pharmaceutically acceptable carrier.

In another preferred embodiment, the dosage form of the pharmaceutical composition is a solid preparation or a liquid preparation.

In another preferred embodiment, the dosage form of the pharmaceutical composition is an oral formulation, an injection, or a topical pharmaceutical formulation.

In another preferred embodiment, the pharmaceutical composition is tablet, granule, or capsule.

In another preferred embodiment, the preparation or the composition comprises 0.001-99wt%, preferably 0.1-90wt%, and more preferably 1-50wt% of the A-decarbonized-5α-androstane compound, based on the total weight of the preparation or the composition.

In another preferred embodiment, the term "increasing the number of white blood cells" means that the number of white blood cells in a subject's blood is increased.

In another preferred embodiment, the subject is human or a non-human mammal (such as rodent).

In another preferred embodiment, the subject is a human with a decreased number of white blood cells.

In another preferred embodiment, the term "decreased number of white blood cells" means that the number A1 of white blood cells in a subject is lower than A0 as compared with the lower limit A0 of the number of white blood cells in the normal human; preferably, A1/A0 is 0.1-0.9 , preferably 0.2-0.8, and more preferably 0.3-0.7.

In another preferred embodiment, the value of A0 is 4x10⁹ cells/L blood.

In another preferred embodiment, the subject is tumor patient.

In another preferred embodiment, the subject is a subject who has been undergone, is, or will be undergoing tumor treatment.

In another preferred embodiment, the tumor treatment comprises chemotherapy, radiotherapy, and a combination thereof.

In another preferred embodiment, the term "increasing the number of white blood cells" means that the number C1 of white blood cells in a subject's blood is increased as compared with the number C0 of white blood cells in the control.

In another preferred embodiment, the number C0 of white blood cells in the control refers to the number (Czs) of white blood cells in the subject before being treated or administered with the medicine of the present invention; or the number (Cdz) of white blood cells in the control group.

In another preferred embodiment, the term "increasing the number of white blood cells" means that C1/Czs is ≥1.2, preferably ≥1.4, and more preferably ≥1.5 or ≥2.0.

In another preferred embodiment, the term "increasing the number of white blood cells" means that C1/Cdz is ≥1.2, preferably ≥1.4, and more preferably ≥1.5 or ≥2.0.

In the second aspect of the present invention, it provides a preparation product for increasing the number of white blood cells or preventing or treating leucopenia, which comprises:
a first pharmaceutical composition, comprising (a) a first active ingredient which is an A-decarbonized-5α-androstane compound; and (b) a pharmaceutically acceptable carrier; and
a second pharmaceutical composition, which is a medicine for increasing the number of white blood cells.

In another preferred embodiment, the medicine for increasing the number of white blood cells comprises (a) a second active ingredient which is an active ingredient for increasing the number of white blood cells and is different from the A-decarbonized-5α-androstane compound; and (b) a pharmaceutically acceptable carrier.

In another preferred embodiment, the term "increasing the number of white blood cells" is to increase the number of white blood cells in a mammal.

In another preferred embodiment, the medicine for increasing the number of white blood cells is selected from the group consisting of Chinese herbal medicine, Chinese medicine compound preparation, chemical medicine, biological preparation, and combinations thereof.

In another preferred embodiment, the Chinese herbal medicine is selected from the group consisting of *Panax ginseng, Astragalus mongholicus, Codonopsis pilosula,* fruit of *Ligustrum lucidum,* stem of *Spatholobus suberectus Dunn,* fruit of *Lycium chinensis, Rehmannia glutinosa (Gaetn.) Libosch,* and combinations thereof.

In another preferred embodiment, the Chinese medicine compound preparation is selected from the group consisting of YuluTang, BaoyuanTang, LiuweiDihuang oral liquid, Shengbai Pill, WujiBaifeng Pill, JianpiYishen Granules, Chang'anShengbai Granules, Shengbai Tablets, Shenqi Tablets, YangxueShengbai Capsules, and combinations thereof.

In another preferred embodiment, the chemical medicine is selected from the group consisting of leucogen, batiloli, vitamin B, berbmine hydrochloride, inosine, DNA, and combinations thereof.

In another preferred embodiment, the biological preparation is selected from the group consisting of colony stimulating factor (CSF), granulocyte colony stimulating factor (G-CSF), granulocyte-macrophage colony stimulating factor (GM-CSF), and combinations thereof.

In another preferred embodiment, the first pharmaceutical composition and the second pharmaceutical composition are independent or combined into one.

In the third aspect of the present invention, it provides a preparation product for treating tumor, which comprises:
(I) a first pharmaceutical composition, comprising (a) a first active ingredient, which is an A-decarbonized-5α-androstane compound; and (b) a pharmaceutically acceptable carrier; and
(II) a second pharmaceutical composition, comprising (a) a second active ingredient which is an active ingredient for treating tumor and is different from the A-decarbonized-5α-androstane compound; and (b) a pharmaceutically acceptable carrier.

In another preferred embodiment, the second active ingredient is selected from the group consisting of cyclophosphamide, ifosfamide, doceflunidine, 5-fluorouracil, cytarabine, fluoroguanosine, tegafur, gemcitabine, carmofur, hydroxyurea, methotrexate, actinomycin D, adriamycin, daunorubicin, epirubicin, mitomycin, penomycin, irinotecan, harringtonine, hydroxycamptothecin, vinorelbine, paclitaxel, taxotere, topotecan, vincristine, vindesine, teniposide, etoposide, atamestane, anastrozole, aminoglutethimide, letrozole, formestane, megestrol, tamoxifen, asparaginase, carboplatin, cisplatin, dacarbazine, oxaliplatin, mitoxantrone, procarbazine, and combinations thereof.

In another preferred embodiment, the first pharmaceutical composition and the second pharmaceutical composition are independent or combined into one.

In the fourth aspect of the present invention, it provides a method for increasing the number of white blood cells, which comprises: (a) administering an A-decarbonized-5α-androstane compound to a subject in need.

In another preferred embodiment, the subject comprises human or non-human mammal, preferably rodent (such as mouse or rat) or primate (such as human).

In another preferred embodiment, the subject is a tumor patient.

In another preferred embodiment, the tumor is selected from the group consisting of gastric cancer, liver cancer, leukemia, kidney cancer, lung cancer, small intestine cancer, bone cancer, prostate cancer, colorectal cancer, breast cancer, colon cancer, prostate cancer, cervical cancer, lymphoma, adrenal tumor, and bladder tumor.

In another preferred embodiment, the subject has been undergone, is or will be undergoing chemotherapy and/or radiotherapy.

In another preferred embodiment, the subject has received chemotherapy or radiotherapy.

In another preferred embodiment, the number of white blood cells of the subject is lower than a lower limit of normal value.

In another preferred embodiment, the subject has received chemotherapy or radiotherapy and the number of white blood cells is close to or lower than the lower limit of normal value.

In another preferred embodiment, when the subject is human, the normal value is 4x10⁹-10x10⁹/L.

In another preferred embodiment, when the subject is a human, the number of white blood cells is 1x10⁹-3.8x10⁹/L before the number of white blood cells are increased by using the method of the present invention.

In another preferred embodiment, when the subject is a mouse, the normal value is 1.8x10³-10.7x10³/ul.

In another preferred embodiment, the method is non-therapeutic.

It should be understood that, in the present invention, each of the technical features specifically described above and below (such as those in the Examples) can be combined with each other, thereby constituting new or preferred technical solutions which need not be redundantly specified again herein.

### EMBODIMENTS FOR CARRYING OUT THE INVENTION

Based on an extensive and intensive research, the inventors have unexpectedly found for the first time that a class of compounds with a special structure, i.e. the A-decarbonized-5α-androstane compound, can very effectively increase the number of white blood cells in mammals with very low side effects. It has been shown in experiments that there is a significant amelioration and improvement of the decreasing number of white blood cells caused by anti-tumor drugs (such as chemotherapy drugs such as cyclophosphamide) or radiotherapy. In addition, the administration of the A-decarbonized-5α-androstane compound before chemotherapy or radiotherapy can prevent the decrease of the number of white blood cells without a significant decrease in a long time. The inventors have completed the present invention based on this discovery.

### Active Ingredient

In the present invention, the active ingredient for increasing the number of white blood cells is the A-decarbonized-5α-androstane compound, or a pharmaceutically acceptable salt, or a prodrug thereof.

The A-decarbonized-5α-androstane compounds are a class of compounds with special structure independently developed by Ruilin Li, et al. The animal efficacy experiments have showed that the A-decarbonized-5α-androstane compounds have a better effect on treating prostatic hyperplasia. In further research, it has found that the A-decarbonized-5α-androstane compounds have significant anti-malignant tumor activity *in vivo* and *in vitro,* and have the advantage of improving animal weight loss while inhibiting tumor proliferation. These compounds can selectively prevent tumor cell division without affecting normal cells, thereby inhibiting the spread of tumor cells.

The inventors have unexpectedly found that the A-decarbonized-5α-androstane compounds can significantly increase the number of white blood cells.

In the present invention, the terms "active ingredient", "active ingredient of the present invention", or "active ingredient for increasing the number of white blood cells in the present invention" can be used interchangeably, and refer to the A-decarbonized-5α-androstane compound, and especially the compound having the following structure: 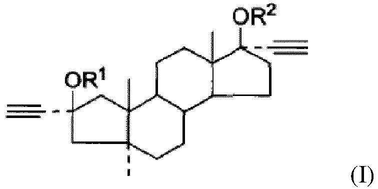 wherein,
R¹ and R² are as defined above.

Preferably, the A-decarbonized-5α-androstane compound is one or more compounds selected from the group consisting of:
2α,17α-diethynyl-A-decarbonized-5α-androstane-2β,17β-dihydroxyl compound (Ia);
2α,17α-diethynyl-A-decarbonized-5α-androstane-2β, 17β-dihydroxyl diacetate compound (Ib);
2α,17α-diethynyl-A-decarbonized-5α-androstane-2β, 17β-dihydroxyl dipropionate compound (Ic);
2α,17α-diacetyl-A-decarbonized-5α-androstane-2β,17β-dihydroxyl-2β-monosuccinate compound (Id);
2α,17α-diethynyl-A-decarbonized-5α-androstane-2β,17β-bissuccinate compound (Ie);
2α,17α-diethynyl-A-decarbonized-5α-androstane-2β,17β-dibutyrate compound (If);
2α,17α-dihydroxylpropynyl-A-decarbonized-5α-androstane-2β,17β-dihydroxyl compound (Ig);
2α,17α-dicyano-A-decarbonized-5α-androstane-2β,17β-dihydroxyl compound (Ih);
2α,17α-diethynyl-A-decarbonized-5α-androstane-2β, 17β-dihydroxyl ditrichloroacetate compound (Ii);
2α,17α-diethynyl-A-decarbonized-5α-androstane-2β,17β-dihydroxyl-2β-propionate-17β-su ccinate compound (Ij);
2α,17α-dipropynyl-A-decarbonized-5α-androstane-2β,17β-dihydroxyl compound (Ik); and/or
2α,17α-dipropynyl-A-decarbonized-5α-androstane-2β, 17β-dihydroxyl dipropionate compound (Il).

### Pharmaceutical Composition

The present invention provides a pharmaceutical composition for increasing the number of white blood cells, which comprises (a) an A-decarbonized-5α-androstane compound; and (b) a pharmaceutically acceptable carrier or a food acceptable carrier.

The "active ingredient" in the pharmaceutical composition of the present invention refers to the compound of formula (I) according to the present invention.

The "active ingredient" and the pharmaceutical composition according to the present invention can be used to increase the number of white blood cells.

In another preferred embodiment, they are used for preparing a medicine for increasing the number of white blood cells.

The term "safe and effective amount" means that the amount of active ingredient is sufficient to significantly improve the condition without causing serious side effects.

Generally, the pharmaceutical composition comprises 1-2000mg of active ingredient/dose, and more preferably 10-200 mg of active ingredient/dose. Preferably, the "one dose" is a tablet or an injection.

The term "pharmaceutically acceptable carrier" refers to one or more compatible solid or liquid fillers or gel substances that are suitable for human use and must have sufficient purity and low enough toxicity.

The term "compatible" as used herein means that each component in the composition can blend with the active ingredient of the present invention and blended with each other without significantly reducing the effect of the active ingredient.

Some examples of pharmaceutically acceptable carriers are cellulose and its derivatives (such as sodium carboxymethyl cellulose, sodium ethyl cellulose, cellulose acetate, etc.), gelatin, talc, solid lubricant (such as stearic acid, magnesium stearate), calcium sulfate, vegetable oil (such as soybean oil, sesame oil, peanut oil, olive oil, etc.), polyol (such as propylene glycol, glycerin, mannitol, sorbitol, etc.), emulsifier (such as Tween®), wetting agent (such as sodium lauryl sulfate), colorant, flavoring agent, stabilizer, antioxidant, preservative, pyrogen-free water, etc.

In another preferred embodiment, the compound of formula (I) in the present invention can form a complex with macromolecular compound or polymer via non-bonding action.

In another preferred example, the compound of formula (I) of the present invention as a small molecule can be linked to a macromolecular compound or polymer via a chemical bond. The macromolecular compound can be biological macromolecule such as polysaccharide, protein, nucleic acid, polypeptide, etc.

There is no particularly limitation to the administration mode of the active ingredient or the pharmaceutical composition in the present invention, and the representative administration modes comprise, but are not limited to, oral, intratumoral, rectal, parenteral (intravenous, intramuscular or subcutaneous) administration and the like.

Solid dose forms for oral administration comprise capsule, tablet, pill, powder and granule.

In these solid dose forms, the active ingredient is mixed with at least one conventional inert excipient or carrier, such as sodium citrate or dicalcium phosphate, or is mixed with one or more of the following ingredients:
(a) fillers or solubilizers, such as starch, lactose, sucrose, glucose, mannitol and silicic acid;
(b)binders, such as hydroxymethyl cellulose, alginates, gelatin, polyvinylpyrrolidone, sucrose, and acacia;
(c) moisturizer, such as glycerin;
(d) disintegrants, such as agar, calcium carbonate, potato starch or cassava starch, alginic acid, some complex silicate, and sodium carbonate;
(e)solvents, such as paraffin;
(f) absorption accelerators, such as quaternary amine compound;
(g) wetting agents, such as cetanol and glyceryl monostearate;
(h) adsorbents, such as kaolin; and/or
(i) lubricants, such as talc, calcium stearate, magnesium stearate, solid poly(ethylene glycol), sodium lauryl sulfate, and a mixture thereof.

In capsule, tablet and pill, the dosage form can also comprise a buffer agent.

The solid dosage form can also be prepared by using coating and shell materials, such as enteric coating and other materials known in the art. They can comprise opaque agents and the active ingredient in the composition can be released in the certain part of the digestive tract in a delayed mode. Examples of useful embedding components are polymeric substance and waxes.

Liquid dosage form for oral administration comprises pharmaceutically acceptable emulsion, solution, suspension, syrup or tincture. In addition to the active ingredient, liquid dosage form can comprise inert diluent conventionally used in the art, such as water or other solvents, solubilizers, and emulsifiers, such as ethanol, isopropanol, ethyl carbonate, ethyl acetate, propylene glycol, 1,3-butanediol, dimethylformamide, and oil, especially cottonseed oil, peanut oil, corn germ oil, olive oil, castor oil, sesame oil, and a mixture thereof. In addition to these inert diluents, the composition can also comprise additives such as wetting agent, emulsifier and suspending agent, sweetener, flavoring agent, and spice.

In addition to the active ingredient, the suspension can comprise a suspending agent, such as ethoxylated isostearyl alcohol, polyoxyethylene sorbitol, dehydrated sorbitan ester, microcrystalline cellulose, aluminum methoxide, agar, and a mixture thereof, and the like.

Compositions for parenteral injection can comprise physiologically acceptable sterile aqueous or anhydrous solution, dispersion, suspension or emulsion, and sterile powders for reconstitution into sterile injectable solution or dispersion. Suitable aqueous and non-aqueous carrier, diluent, solvent or excipient comprise water, ethanol, polyols and suitable mixtures thereof.

When a pharmaceutical composition is used, a safe and effective amount of the compound of the present invention is administered to a mammal (such as human) in need of treatment, wherein the dose of administration is a pharmaceutically effective dose. For a human with 60 kg body weight, the daily administration dose is usually 1-1000mg, preferably 10-200mg, and more preferably 20-100mg. The specific dose should also consider factors such as the route of administration, the patient's health status, etc., which are all within the skills of an experienced physician.

The compound of the present invention can be administered alone or in combination with other therapeutic medicines (especially anticancer medicines or medicines for increasing white cells).

The compound of formula (I) can also be used in combination with other medicines known to treat or improve similar condition. When combined, the administration mode and dose of the original medicine remain unchanged, while the compound of formula (I) is administered simultaneously or subsequently. When the compound of formula (I) is administered simultaneously with one or more other medicines, it is preferred to use a pharmaceutical composition containing one or several known medicines and the compound of formula (I). Co-administration also comprises administration of the compound of formula (I) with one or more other known medicines over an overlapping period of time. When the compound of formula (I) is used in combination with one or more other medicines, the dose of the compound of formula (I) or known medicine can be lower than a dose for administration of each of them alone.

### Oral Preparation

The invention provides an oral preparation with the A-decarbonized-5α-androstane compound as an active ingredient, and a pre-prescription research has been carried out to provide a basis for studying the pharmaceutical preparation.

The pre-prescription research results have showed that the water-insoluble A-decarbonized-5α-androstane compound (the solubility in water is 6.5µg/ml, which is defined as insoluble according to the pharmacopoeia) has good dissolution and absorption in intestinal fluid. The mechanisms of transport, uptake and absorption are all passive diffusion, and amount of uptake is time-dependent and dose-dependent. The A-decarbonized-5α-androstane compound can be administered by oral route. The treatment of cancer requires long-term administration, the oral administration can improve patient compliance, and thus the dosage form is designed as oral preparation, such as common oral tablet, oral capsule, and other sustained-release preparation.

The components and weight contents thereof in an oral preparation of the invention are as follows:

| Preparation Components | Parts by weight |
|---|---|
| Active ingredient | 1 - 50 |
| Filler | 20 - 95 |
| Disintegrant | 0 - 20 |
| binder | 0.1 - 30 |
| Lubricant | 0.1 - 5 |
| glidant | 0.1-5 |

The active component is the A-decarbonized-5α-androstane compound.

The filler added can be one or more ingredients that increase the weight and volume of tablet. In the present invention, the filler is one or more substances selected from lactose, sucrose, sorbitol, mannitol, polyethylene glycol, starch, and inorganic salts. The amount of filler is 20-95%, preferably 60-95%, more preferably 70-95%, and most preferably 80-95% of the total weight of preparation. When lactose is used as a filler, the amount of lactose is 20-95% of the total weight of preparation. When sucrose is used as a filler, the amount of sucrose is 10-30% of the total weight of preparation. When mannitol is used as a filler, the amount of mannitol is 20-95% of the total weight of preparation. When inorganic salt is used as a filler, the amount of inorganic salt is 5-20 % of the total weight of preparation. In another preferred embodiment, the filler is lactose, mannitol, sorbitol, and a mixture thereof; preferably, the filler is a mixture of lactose and mannitol.

The disintegrant is selected from the group consisting of crospovidone (PVPP), croscarmellose sodium (CCNa), sodium carboxymethyl starch (CMS-Na), and low-substituted hydroxypropyl cellulose (L-HPC) and combinations thereof. The PVPP and CCNa are preferred, and CCNa is most preferred. The amount of disintegrant is 0-20% (based on the total weight), and the general amount is 1-10%, and most preferably 3-5%.

The lubricant is one or two or more substances selected from stearic acid, sodium stearate, magnesium stearate, calcium stearate, poly(ethylene glycol), and hydrogenated vegetable oil. Among them, magnesium stearate is most suitable. The amount of lubricant is 0.1-5% (based on total weight), and the general amount is 0.2-4%, and most preferably 0.3-3%.

The binder used can be one or more ingredients which are advantageous for granulation. The binder is one or more substances selected from starch syrup, hydroxypropyl methylcellulose (HPMC), polyethylene glycol, povidone (PVP) or copovidone (Kollidon). PVP is preferred. When starch syrup is used as a binder, the amount of starch syrup is 10-30% of the total weight of preparation. When HPMC is used as a binder, the amount of HPMC is 2-5% of the total weight of preparation. When PVP is used as a binder, the amount of PVP is 2-20% of the total weight of preparation. When copovidone is used as a binder, the amount of copovidone is 0.1-10% of the total weight of preparation.

The glidant is one or two substances selected from colloidal silicon dioxide and talc, and more preferably is colloidal silicon dioxide.

In another preferred embodiment, the inorganic salt is selected from the group consisting of calcium sulfate containing two molecules of crystal water; calcium hydrogen phosphate; medical calcium carbonate, etc.

Generally, the tablet of the present invention can also comprises other excipients that are well known to those skilled in the art, and the tablet of the present invention can be prepared by using well-known preparation techniques in the art. For example, direct compression, wet granulation compression and dry granulation compression can be used to prepare tablet.

The present invention provides a common tablet containing the A-decarbonized-5α-androstane compound, and its clinical administration dose can be determined according to pharmacodynamic study to meet the needs of clinical treatment, and the dosage of the A-decarbonized-5α-androstane compound in the tablets is 1-100 mg/tablet, preferably 1-50 mg/tablet, such as 2.5 mg/tablet, 5 mg/tablet, 10 mg/tablet, 25 mg/tablet, etc.

### The main advantages of the present invention include:

(a) The active ingredient for increasing white blood cells (i.e. the A-decarbonized-5α-androstane compound) can very effectively increase the number of white blood cells in mammal.
(b) The side effects of the active ingredient for increasing the number of white blood cells are very low.
(c) The active ingredient for increasing the number of white blood cells does not cause excessive increase in the number of white blood cells (i.e., does not exceed the upper limit of normal value), and the effect of increasing the number of white blood cells can be maintained for a long time without significant decrease.
(d) The active ingredient of the present invention can be used not only for the treatment of leukopenia, but also for the prevention of leukopenia.
(e) Combination of the active ingredient of the present invention and other tumor treatment methods (such as chemotherapy) can further reduce the side effects of other chemotherapeutics and enhance the therapeutic effect, and has a synergistic effect.

The present invention will be further illustrated below with reference to the specific examples. It should be understood that these examples are only to illustrate the invention but not to limit the scope of the invention. The experimental methods with no specific conditions described in the following examples are generally performed under the conventional conditions, such as Sambrook et al., Molecular Cloning: Laboratory Manual (New York: Cold Spring Harbor Laboratory Press, 1989), or according to the manufacturer's instructions.

### Materials

ACP1: 2α,17α-diethynyl-A-decarbonized-5α-androstane-2β,17β-dihydroxyl diacetate compound (Ib);
ACP2:
   2α,17α-diacetyl-A-decarbonized-5α-androstane-2β, 17β-dihydroxyl-2β-monosuccinate compound (Id);
ACP3: 2α,17α-diethynyl-A-decarbonized-5α-androstane-2β,17β-dibutyrate compound (If);
ACP4: 2α,17α-dipropynyl-A-decarbonized-5α-androstane-2β,17β-dihydroxyl compound (Ik);
ACP5: 2α,17α-dipropynyl-A-decarbonized-5α-androstane-2β,17β-dihydroxyl dipropionate compound (Il).

### Example 1 Effect of ACP on increasing the number of white blood cells in Lewis mice with decreased number of white blood cells caused by cyclophosphamide (CTX)

### 1.1 Effect of ACP1 on increasing white blood cells

The vigorously growing tumor cells were taken and prepared into cell suspension with a concentration of about 5-10x10⁶/ml by homogenization under sterile condition.

Mice were inoculated with Lewis lung tumor cells to produce a transplantable tumor model and randomly divided into groups, each group had 10 mice, and the compound for increasing white blood cells was administered on the day of tumor inoculation. Except for high-dose ACP1 administration group, other groups were administered CTX on the 3rd day (150mg/kg) and the 5th day (100mg/kg), thereby establishing an inhibitory tumor-bearing mice model having leukopenia induced by chemotherapy.

At the beginning of the experiment, 10 mice were randomly sampled for blood collection by posterior orbital venous plexus puncture, and an automatic blood cell analyzer (HEMAVET-950) was used to conduct the routine blood test. Then the blood was collected for the routine blood test by the same method on the 8th and 12th days after the last administration of cyclophosphamide.

The results of ACP1 were shown in Table 1.

**Table 1 the experiment of ACP1 combined with cyclophosphamide on increasing white blood cells in mice bearing Lewis lung tumor**

| **Group** | **Dose** | **Administ ration mode** | **Day 8 (10³ WBC/µl)** | **Day 8** (%) | **Day 12 (10³ WBC/µl)** | **Day 12** (%) |
|---|---|---|---|---|---|---|
| Chemotherapy control group CTX | 150/100 mg/kg | ipxqd | 0.41±0.07 | 100% | 1.48±0.37 | 100% |
| High dose of ACP1+CTX | 5mg/kg | igx12 Bid | 1.63±0.79 | 398%* | 2.39±0.95 | 161%* |
| Medium dose of ACP1 +CTX | 2.5mg/kg | igx12 Bid | 1.37±0.24 | 334%* | 2.72±0.92 | 184%* |
| Low dose of ACP1+CTX | 1.25mg/kg | igx12 Bid | 2.66±0.71 | 649%* | 2.95±0.96 | 199%* |
| High dose of ACP alone | 5mg/kg | igx12 Bid | 4.21±1.22 | 1027%* | 4.32±1.29 | 292%* |

| | | | | | | |
|---|---|---|---|---|---|---|
| Note: Compared with the chemotherapy control group: * P <0.05; (Literature data: normal value of white blood cells in mice is 1.8x10³-10.7x10³/µl) | | | | | | |

### Results:

In the chemotherapy control group, cyclophosphamide or CTX caused a decrease of white blood cells of peripheral blood in mice, indicating that the animal model of leukopenia was successfully established.

In the ACP administration group, the effect of increasing white blood cells was statistically significant on both Day 8 and Day 12. Compared with the chemotherapy control group, the extent of increasing white blood cells was about 200% to about 500% on Day 8 and about 60% to about 100% on Day 12. It was showed that ACP1 has a significant effect to improve or resist the cyclophosphamide-induced reduction of the number of white blood cells in mice.

### 1.2 Effect of ACP2-5 on increasing white blood cells

The method was similar to that in Example 1.1, and the differences were as follows: each group had 5 mice; and in the experimental group, ACP1 was replaced with ACP2, ACP3, ACP4 and ACP5, respectively, and only medium dose of ACP + CTX 2.5mg/kg was used. The chemotherapy control group was the same as the chemotherapy control group in Example 1.1. On the 10th day (Day 10), blood was collected and routine blood test was conducted in the same method.

The experimental results showed that in the chemotherapy control group, cyclophosphamide CTX caused a decrease of white blood cells of peripheral blood in mice, indicating that the animal model of leukopenia was successfully established.

In each ACP administration group, the effect of increasing white blood cells was statistically significant on Day 10. Compared with the chemotherapy control group, the extent of increasing white blood cells among ACP2, ACP3, ACP4 and ACP5 was comparable, the extent of increasing white blood cells (Cl/Cdz-100%) was 70% to 240% on Day 10. It was showed that the A-decarbonized-5α-androstane compounds had a significant effect to improve or resist cyclophosphamide-induced reduction of the number of white blood cells in mice.

The above experimental results had showed that the active ingredient for increasing the number of white blood cells in the present invention could significantly alleviate and improve the decrease of the number of white blood cells caused by antitumor drugs (such as cyclophosphamide), and thus can be used for preventing and/or treating leukopenia.

### Example 2 Test of ACP1 on increasing white blood cells in tumor patients

10 cancer patients who had received chemotherapy (adriamycin, cyclophosphamide, irinotecan, paclitaxel, cisplatin, oxaliplatin, vincristine, fluorouracil, etc.) and had a decrease in the number of white blood cells in the peripheral blood were selected as subjects. Blood was collected on the day before the administration, and the routine blood test was conducted to determine the number of white blood cells (Czs) before the subjects were treated with the medicine of the present invention.

10 subjects took ACP at different administrated frequency and dose every day (see Table 2). Blood was collected from 10 subjects at Day 8, 15, 22, and 28 for routine blood test.

**Table 2 Effect of ACP1 on increasing white blood cells in tumor patients**

| **Subject No.** | **Gender** | **WBC count before administration (Day 0)(10⁹/L)** | **White blood cell (WBC) count during administration(10**⁹**/L)** | | | | **Administration mode** | **Total daily dose** |
|---|---|---|---|---|---|---|---|---|
| | | | **Day 8** | **Day 15** | **Day 22** | **Day 28** | | |
| GBYI | male | 4.3 | 5.8 | 6.9 | 6.3 | 7.0 | Once a day | 15mg |
| YYSO | male | 4.3 | 5.6 | 5.5 | 6.1 | 4.6 | Once a day | 15mg |
| PLSH | male | 3 | 4.5 | 4.9 | 5.0 | 4.7 | Three times a day | 15mg |
| SLYI | female | 4.9 | 6.2 | 8.1 | 6.6 | 6.4 | Three times a day | 30mg |
| ZYZH | female | 4.6 | 5.6 | 5.8 | 5.0 | 4.1 | Three times a day | 30mg |
| LXJV | female | 2.7 | 5.9 | 7.5 | 6.0 | 6.1 | Three times a day | 30mg |
| WZZH | male | 4 | 5.6 | 9.4 | 8.8 | 7.8 | Three times a day | 30mg |
| YSMI | male | 3.8 | 3.8 | 4.9 | 4.7 | 4.7 | Three times a day | 45mg |
| JYFA | female | 3.8 | 7.3 | 9.7 | 7.8 | 5.2 | Three times a day | 45mg |
| FSPI | female | 3.6 | 5.4 | 5.8 | 5.7 | 5.9 | Three times a day | 45mg |
| Effect of increasing WBC (Cl/Czs) | | 100% | 146% | 180% | 163% | 149% | | |

| | | | | | | | | |
|---|---|---|---|---|---|---|---|---|
| Note: normal value of white blood cell (WBC) count is 4x10⁹-10x10⁹/L | | | | | | | | |

The results showed that ACP1 could significantly increase the number of white blood cells.
(i) On the 8th day (Day 8) after administration, the white blood cell count (C1/Czs) in most subjects were increased significantly (the extent of increasing white blood cells (C1/Czs-100%)was about 50% compared with that before treatment with the medicine for increasing blood cells).
(ii) On the 15th day (Day 15) after administration, the white blood cell count (C1/Czs) in the subjects was further increased, with an average increasing extent of about 80% and a maximum of about 178%.
(iii) On Day 22 after administration, the extent of increasing white blood cells in subjects was about 63%.
(iv) On day 28 after administration, the average extent of increasing white blood cells in subjects was about 50%. During the entire 28-day test period, the subject's white blood cell count was not decreased significantly and maintained at normal white blood cell level.

Therefore, the compound of the present invention could significantly increase the number of white blood cells in tumor patient and had a significant effect of increasing white blood cells.

The example showed that for the subjects who had been treated with anti-tumor medicines for chemotherapy and resulted in a decrease in the number of white blood cells (close to or below the lower limit of the normal WBC value), the active ingredient for increasing white blood cells in the present invention had a significant improvement effect and could be used for treat leukopenia caused by chemotherapy (or radiotherapy).

All literatures mentioned in the present application are incorporated herein by reference, as though each one is individually incorporated by reference. Additionally, it should be understood that after reading the above teachings, those skilled in the art can make various changes and modifications to the present invention. These equivalents also fall within the scope defined by the appended claims.

## Claims

1. A use of an A-decarbonized-5α-androstane compound for manufacturing a preparation or a composition, wherein the preparation or the composition is used for (a) increasing the number of white blood cells; or (b) preventing or treating leucopenia.

2. The use of claim 1, wherein the A-decarbonized-5α-androstane compound has a structure of formula I: 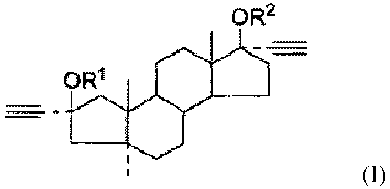 wherein,
R¹ and R² are independently selected from the group consisting of H, substituted or unsubstituted C₁₋₁₀ alkyl, substituted or unsubstituted C₃₋₈ cycloalkyl, substituted or unsubstituted benzene ring, substituted or unsubstituted benzoyl, substituted or unsubstituted COCₙH₂ₙ₊₁, substituted or unsubstituted COCᵣH₂ᵣCOOCₘH₂ₘ₊₁, or -COCₚH₂ₚCOO-W; wherein each of n, p, r, and m is independently an integer from 0 to 18, W is H, Na⁺, K⁺, NH₄⁺, 1/2Ca²⁺, 1/2Mg²⁺, 1/2(AlOH)²⁺ or 1/2Zn²⁺,
the term "substituted" means a substitution with one or more (such as 1-3) substituents selected from the group consisting of hydroxyl, halogen, nitro, amino, amine and carboxyl.

3. The use of claim 1, wherein the A-decarbonized-5α-androstane compound is selected from the group consisting of:
2α,17α-diethynyl-A-decarbonized-5α-androstane-2β,17β-dihydroxyl compound;
2α,17α-diethynyl-A-decarbonized-5α-androstane-2β, 17β-dihydroxyl diacetate compound;
2α,17α-diethynyl-A-decarbonized-5α-androstane-2β, 17β-dihydroxyl dipropionate compound;
2α,17α-diacetyl-A-decarbonized-5α-androstane-2β, 17β-dihydroxyl-2β-monosuccinate compound;
2α,17α-diethynyl-A-decarbonized-5α-androstane-2β,17β-bissuccinate compound;
2α,17α-diethynyl-A-decarbonized-5α-androstane-2β,17β-dibutyrate compound;
2α,17α-dihydroxylpropynyl-A-decarbonized-5α-androstane-2β,17β-dihydroxyl compound;
2α,17α-dicyano-A-decarbonized-5α-androstane-2β,17β-dihydroxyl compound;
2α,17α-diethynyl-A-decarbonized-5α-androstane-2β, 17β-dihydroxyl ditrichloroacetate compound;
2α,17α-diethynyl-A-decarbonized-5α-androstane-2β,17β-dihydroxyl-2β-propionate-17β-su ccinate compound;
2α,17α-dipropynyl-A-decarbonized-5α-androstane-2β,17β-dihydroxyl compound; and
2α,17α-dipropynyl-A-decarbonized-5α-androstane-2β, 17β-dihydroxyl dipropionate compound.

4. The use of claim 1, wherein the preparation or the composition comprises 0.001-99 wt%, preferably 0.1-90 wt%, and more preferably 1-50 wt% of the A-decarbonized-5α-androstane compound, based on the total weight of the preparation or the composition.

5. A preparation product for increasing the number of white blood cells or preventing or treating leucopenia, which comprises:
a first pharmaceutical composition, comprising (a) a first active ingredient which is an A-decarbonized-5α-androstane compound; and (b) a pharmaceutically acceptable carrier; and
a second pharmaceutical composition, which is a medicine for increasing the number of white blood cells.

6. The preparation product of claim 5, wherein the medicine for increasing the number of white blood cells comprises (a) a second active ingredient, which is an active ingredient for increasing the number of white blood cells and is different from the A-decarbonized-5α-androstane compound; and (b) a pharmaceutically acceptable carrier.

7. The preparation product of claim 5, wherein the medicine for increasing the number of white blood cells is selected from the group consisting of Chinese herbal medicine, Chinese medicine compound preparation, chemical medicine, biological preparation, and combinations thereof.

8. A preparation product for treating tumor, which comprises:
(I) a first pharmaceutical composition, comprising (a) a first active ingredient, which is an A-decarbonized-5α-androstane compound; and (b) a pharmaceutically acceptable carrier; and
(II) a second pharmaceutical composition, comprising (a) a second active ingredient, which is a active ingredient for treating tumor and is different from the A-decarbonized-5α-androstane compound; and (b) a pharmaceutically acceptable carrier.

9. The preparation product of claim 8, wherein the second active ingredient is selected from the group consisting of cyclophosphamide, ifosfamide, doceflunidine, 5-fluorouracil, cytarabine, fluoroguanosine, tegafur, gemcitabine, carmofur, hydroxyurea, methotrexate, actinomycin D, adriamycin, daunorubicin, epirubicin, mitomycin, penomycin, irinotecan, harringtonine, hydroxycamptothecin, vinorelbine, paclitaxel, taxotere, topotecan, vincristine, vindesine, teniposide, etoposide, atamestane, anastrozole, aminoglutethimide, letrozole, formestane, megestrol, tamoxifen, asparaginase, carboplatin, cisplatin, dacarbazine, oxaliplatin, mitoxantrone, procarbazine, and combinations thereof.

10. A method for increasing the number of white blood cells, which comprises: (a) administering an A-decarbonized-5α-androstane compound to a subject in need.
